(19) Europäisches Patentamt
European Patent Office
Office européen des brevets

(11) Veröffentlichungsnummer: **0 007 986**
**B1**

(12) # EUROPÄISCHE PATENTSCHRIFT

(45) Veröffentlichungstag der Patentschrift: **16.09.81**

(51) Int. Cl.³: **C 07 D 401/12,** A 61 K 31/415, A 61 K 31/44

(21) Anmeldenummer: **79101924.3**

(22) Anmeldetag: **13.06.79**

(54) **Neue substituierte 2-Phenylamino-imidazoline-(2), deren Säureadditionssalze, diese enthaltende Arzneimittel und Verfahren zur Herstellung derselben.**

(30) Priorität: **15.07.78 DE 2831143**

(43) Veröffentlichungstag der Anmeldung: **20.02.80 Patentblatt 80/4**

(45) Bekanntmachung des Hinweises auf die Patenterteilung: **16.09.81 Patentblatt 81/37**

(84) Benannte Vertragsstaaten: **AT BE CH DE FR GB IT LU NL SE**

(56) Entgegenhaltungen:
**DE-A-1 957 722**
**DE-A-2 220 906**

(73) Patentinhaber: **C.H. BOEHRINGER SOHN, Postfach 200, D-6507 Ingelheim am Rhein (DE)**

(72) Erfinder: **Stähle, Helmut, Dr., Rotweinstrasse 23, D-6507 Ingelheim am Rhein (DE)**
Erfinder: **Köppe, Herbert, Dr., Neuweg 72, D-6507 Ingelheim am Rhein (DE)**
Erfinder: **Kummer, Werner, Dr., Georg-Scheuing-Strasse 15, D-6507 Ingelheim/Rhein (DE)**
Erfinder: **Hoefke, Wolfgang, Dr., Schillerstrasse 13, D-6501 Budenheim (DE)**

**EP 0 007 986 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue substituierte 2-Phenylamino-imidazoline-(2) deren Säureadditionssalze, diese enthaltende Arzneimittel und Verfahren zur Herstellung derselben

Die Erfindung betrifft neue substituierte 2-Phenylamino-imidazoline-(2) der allgemeinen Formel

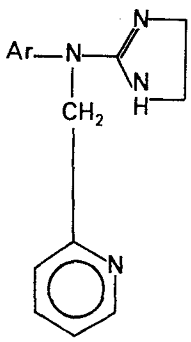

I

sowie deren physiologisch verträgliche Säureadditionssalze mit wertvollen therapeutischen Eigenschaften.

In der Formel I bedeutet Ar den Rest 2,6-Dichlorphenyl, 2,5-Dichlorphenyl, 2-Chlor-6-methylphenyl, 2-Chlor-4-methylphenyl, 2,6-Dichlor-4-bromphenyl, 4-Cyanophenyl, 2,5-Dimethoxyphenyl oder 2-Methyl-5-fluorphenyl.

Die Herstellung der Verbindungen der Formel I erfolgt durch:

a) Umsetzung eines 2-Phenylimino-imidazolidins der allgemeinen Formel

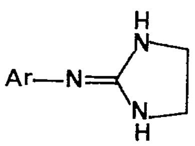

II

in der Ar die oben genannten Bedeutungen besitzt, mit einem Halogenid der allgemeinen Formel

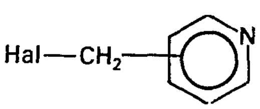

III

worin Hal ein Chlor-, Brom- oder Jodatom bedeutet; oder

b) Umsetzung einer Verbindung der allgemeinen Formel

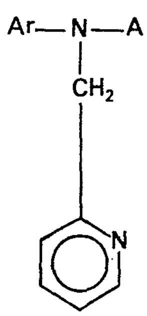

IV

in der Ar wie oben angegeben definiert ist und

A eine Cyanogruppe oder den Rest

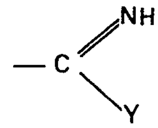

bedeutet, wobei Y eine Alkoxy- oder Alkylthiogruppe mit bis zu 4 C-Atomen oder eine Sulfhydryl- oder Aminogruppe darstellt, mit Äthylendiamin oder dessen Säureadditionssalzen.

Bei der Alkylierung der 2-Arylimino-imidazolidine der Formel II nach Verfahren a) erfolgt die Substitution ausschliesslich am Brückenstickstoffatom. Bei der Umsetzung nach den Verfahren b) ist die Konstitution der Endverbindung durch die Synthese festgelegt. Die Position der Substituenten lässt sich ausser durch die Synthese auch durch die NMR-Spektroskopie festlegen. (Vgl. H. Stähle und K.-H. Pook, Liebigs Ann. Chem. 751, 159 ff (1971).

Die Umsetzung nach Verfahren a) erfolgt zweckmässigerweise durch Erhitzen der Reaktionspartner – vorzugsweise in Gegenwart eines polaren oder unpolaren organischen Lösungsmittels – auf Temperaturen von etwa 50 bis 150 °C. Die speziellen Reaktionsbedingungen hängen in starkem Masse von der Reaktivität der Umsetzungsteilnehmer ab. Es empfiehlt sich, bei der Alkylierung das Halogenid im Überschuss anzuwenden und die Umsetzung in Gegenwart eines säurebindenden Mittels durchzuführen.

Bei dem Verfahren nach b) ist es erforderlich, bei erhöhter Temperatur, zwischen 60 °C und 180 °C zu arbeiten. Lösungsmittel sind nicht erforderlich. Es ist zweckmässig, das als Reaktionspartner verwendete Äthylendiamin bzw. dessen Säureadditionssalz, im Überschuss anzuwenden.

Ausgangsverbindungen der Formel II sind z.B. in den belgischen Patenten Nr. 623 305, 687 657 und 705 944 beschrieben.

Ausgangsverbindungen der Formel III lassen sich durch Halogenierung der zugrundeliegenden primären Alkohole darstellen.

Verbindungen der Formel IV erhält man ausgehend von Anilinen, durch Umsetzung mit Verbindungen der Formel III und nachfolgende Reaktion der dabei entstehenden sekundären Amine, mit Cyanaten oder Thiocyanaten wobei Harnstoffe, bzw. Thioharnstoffe gebildet werden. Harnstoffe und Thioharnstoffe können dann weiter mit Alkylierungsmitteln in entsprechende Isouroniumsalze bzw. Isothiouroniumsalze überführt werden. Aus diesen Säureadditionsverbindungen lassen sich mit Basen die entsprechenden Isoharnstoffe bzw. Isothioharnstoffe gewinnen. Durch Wasserabspaltung aus Harnstoffen bzw. $H_2S$-Abspaltung aus Thioharnstoffen mittels Blei- oder Quecksilbersalzen, gelangt man zu Cyanamiden, an die Ammoniak unter Bildung von Guanidinen angelagert werden kann.

Die erfindungsgemässen 2-Phenylamino-imidazoline-(2) der allgemeinen Formel I können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Die neuen Verbindungen der allgemeinen Formel I und deren Säureadditionssalze wirken sehr stark bradycard und sind daher zur Therapie von Coronarerkrankungen geeignet. Die Beeinflussung der Herzfrequenz wurde an Kaninchen und an Spinalratten sowie an intakten, narkotisierten Ratten untersucht.

Die Dosierung liegt bei 0,1 bis 80 mg, vorzugsweise 1 bis 30 mg.

Die Verbindungen der Formel I bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung, ohne sie zu beschränken.

**Beispiel 1**

2-[N-(2,6-Dichlorphenyl)-N-(α-picolyl)-amino]-2-imidazolin

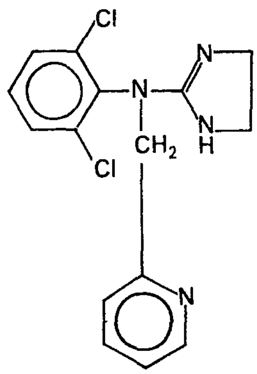

4,6 g (0,02 Mol) 2-(2,6-Dichlorphenylimino)-imidazolidin werden zusammen mit 4,9 g (150%) α-Picolylchlorid-Hydrochlorid in 50 ml Äthylenglykolmonomethyläther unter Zugabe von 4,3 g Kaliumcarbonat 5 Stunden lang unter Rührung am Rückfluss erhitzt. Hierauf wird im Vakuum das Lösungsmittel abdestilliert und der Rückstand in 1 N Salzsäure gelöst. Die salzsaure Lösung wird sodann mit Äther überschichtet und unter guter Rührung mit 50%iger Kalilauge alkalisiert und die ausgeschiedene Rohsubstanz abgesaugt. Zur Reinigung wird diese wiederum in 1 N Salzsäure gelöst, und die salzsaure Lösung bei aufsteigenden pH-Werten (stufenweises Alkalisieren mit 2 N Natronlauge) zunächst fraktioniert Äther extrahiert und dann ab pH 8,5 fraktioniert gefällt und abgesaugt. (Ätherextrakte werden verworfen.) Die festen Fraktionen werden vereint, mit Wasser gewaschen und getrocknet.

Ausbeute: 1,5 g (23,4% der Theorie), Schmelzpunkt: 171–172 °C

Rf: 0,4, System: Benzol 50, Dioxan 40, Äthanol 5, k. $NH_4OH$ 5 Träger: Kieselgel-G-Leuchtpigment, Detektor: UV und Kaliumjodplatinat, $C_{15}H_{14}Cl_2N_4$ (321,22)

| | C | H | Cl | N |
|---|---|---|---|---|
| Ber.: | 56,10% | 4,39% | 22,10% | 17,45% |
| Gef.: | 56,11% | 4,55% | 21,62% | 17,01% |

Analog dem vorstehenden Beispiel wurden die folgenden Verbindungen hergestellt. Die Schmelzpunkte beziehen sich auf die Basen der Formel I.

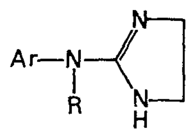

| Beispiel Nr. | Ar | R | Fp. °C | Ausbeute |
|---|---|---|---|---|
| 2 | 2,6-Dichlorphenyl | 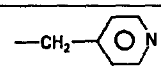 | 182–183 | 34,4 |
| 3 | 2,6-Dichlorphenyl | 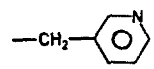 | 143–145 | 28,1 |
| 4 | 2,5-Dichlorphenyl | 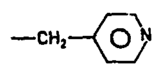 | 128–129 | 11,7 |
| 5 | 2-Chlor-6-methylphenyl | 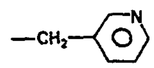 | Öl | 20,0 |
| 6 | 2-Chlor-4-methylphenyl | 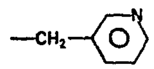 | Öl | 18,4 |
| 7 | 2,6-Dichlor-4-brom-phenyl | 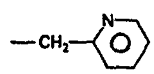 | 139–141 | 25,0 |
| 8 | 4-Cyanophenyl | 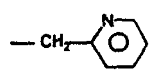 | 128–129 | 23,1 |
| 9 | 2,5-Dimethoxyphenyl | 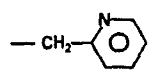 | 129–130 | 33,6 |
| 10 | 2-Methyl-5-fluorphenyl | 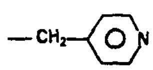 | 121–123 | 32,4 |

Formulierungsbeispiele

Beispiel A: Dragées

| | | |
|---|---|---|
| Wirkstoff gemäss Erfindung | | 5 mg |
| Milchzucker | | 65 mg |
| Maisstärke | | 130 mg |
| sek. Calciumphosphat | | 40 mg |
| lösliche Stärke | | 3 mg |
| Magnesiumstearat | | 3 mg |
| kolloidale Kieselsäure | | 4 mg |
| | insgesamt | 250 mg |

Herstellung:
Der Wirkstoff wird mit einem Teil der Hilfsstoffe vermischt, intensiv mit einer wässrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht gepresst, die dann in üblicher Weise mit Hilfe von Zucker, Talkum und Gummi arabicum dragiert werden.

Beispiel B: Ampullen

| | |
|---|---|
| Wirkstoff gemäss Erfindung | 1,0 mg |
| Natriumchlorid | 18,0 mg |
| dest. Wasser | ad 2,0 mg |

Herstellung:
Wirkstoff und Natriumchlorid werden in Wasser gelöst und unter Stickstoff in Glasampullen abgefüllt.

Beispiel C: Tropfen

| | | |
|---|---|---|
| Wirkstoff gemäss Erfindung | | 0,02 g |
| p-Hydroxybenzoesäuremethylester | | 0,07 g |
| p-Hydroxybenzoesäurepropylester | | 0,03 g |
| entmineralisiertes Wasser | ad | 100 ml |

**Patentansprüche:**

*für die Vertragsstaaten BE, CH, DE, FR, GB, IT, LU, NL, SE*

1. Substituierte 2-Phenylamino-imidazoline-(2) der allgemeinen Formel

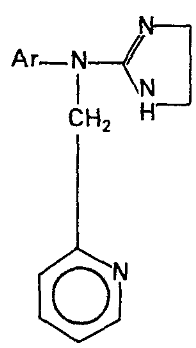

I

worin Ar den Rest 2,6-Dichlorphenyl, 2,5-Dichlorphenyl, 2-Chlor-6-methylphenyl, 2-Chlor-4-methylphenyl, 2,6-Dichlor-4-bromphenyl, 4-Cyanophenyl, 2,5-Dimethoxyphenyl oder 2-Methyl-5-fluorphenyl bedeutet sowie deren Säureadditionssalze.

2. Verfahren zur Herstellung von substituierten 2-Phenylamino-imidazolinen-(2) der allgemeinen Formel I nach Anspruch 1 und von deren Säureadditionssalzen, dadurch gekennzeichnet, dass man

a) ein 2-Phenyl-iminoimidazolidin der allgemeinen Formel

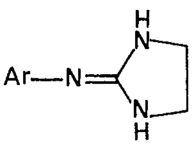

II

in der Ar die oben genannten Bedeutungen besitzt, mit einem Halogenid der allgemeinen Formel

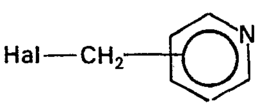

III

worin Hal ein Chlor-, Brom- oder Jodatom bedeutet, umsetzt oder

b) eine Verbindung der allgemeinen Formel

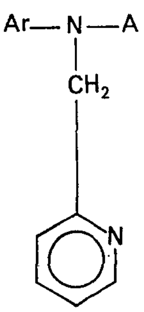

IV

in der Ar wie oben angegeben definiert ist und A eine Cyanogruppe oder den Rest

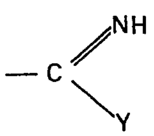

bedeutet, wobei Y eine Alkoxy- oder Alkylthiogruppe mit bis zu 4 Kohlenstoffatomen oder eine Sulfhydryl- oder Aminogruppe darstellt, mit Äthylendiamin oder dessen Säureadditionssalzen umsetzt, und dass man gegebenenfalls das nach einem der Verfahren erhaltene Endprodukt in ein Säureadditionssalz überführt.

3. Verfahren nach Anspruch 2 a), dadurch gekennzeichnet, dass man die Umsetzung von Temperaturen von etwa 50 bis 150 °C durchführt.

4. Verfahren nach Anspruch 2 b), dadurch gekennzeichnet, dass man die Umsetzung bei einer Temperatur zwischen 60 und 180 °C durchführt.

5. Verfahren nach Anspruch 2 a) und/oder 3, dadurch gekennzeichnet, dass man die Umsetzung in Gegenwart eines polaren oder unpolaren organischen Lösungsmittels durchführt.

6. Verfahren nach Anspruch 2 a), 3 und/oder 5, dadurch gekennzeichnet, dass man die Umsetzung in Gegenwart eines säurebindenden Mittels durchführt.

7. Pharmazeutische Zubereitungen, dadurch gekennzeichnet, dass sie als Wirkstoff eine oder mehrere Verbindungen der allgemeinen Formel I nach Anspruch 1 oder deren Säureadditionssalze enthalten.

8. Verfahren zur Herstellung von Arzneimitteln nach Anspruch 7, dadurch gekennzeichnet, dass man eine oder mehrere Verbindungen der allgemeinen Formel I nach Anspruch 1 oder deren Säureadditionssalze mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

9. Verbindungen der allgemeinen Formel I nach Anspruch 1 oder deren physiologisch verträgliche Säureadditionssalze zur Verwendung bei der Behandlung von Herz- und Coronarerkrankungen.

10. Verwendung von Verbindungen der allgemeinen Formel I nach Anspruch 1 oder von deren physiologisch verträglichen Säureadditionssalzen zur Herstellung von Arzneimitteln für die Behandlung von Herz- und Coronarerkrankungen.

**Patentansprüche:**

*für den Vertragsstaat AT*

1. Verfahren zur Herstellung von substituierten 2-Phenylamino-imidazolinen-(2) der allgemeinen Formel

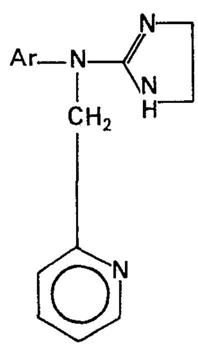

I

worin Ar den Rest 2,6-Dichlorphenyl, 2,5-Dichlorphenyl, 2-Chlor-6-methylphenyl, 2-Chlor-4-methylphenyl, 2,6-Dichlor-4-bromphenyl, 4-Cyanophenyl, 2,5-Dimethoxyphenyl oder 2-Methyl-5-fluorphenyl bedeutet, und von deren Säureadditionssalzen, dadurch gekennzeichnet, dass man

a) ein 2-Phenyl-iminoimidazolidin der allgemeinen Formel

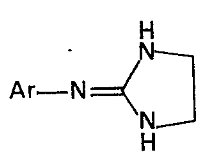

II

in der Ar die oben genannten Bedeutungen besitzt, mit einem Halogenid der allgemeinen Formel

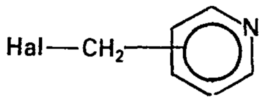

III

worin Hal ein Chlor-, Brom- oder Jodatom bedeutet, umsetzt oder

b) eine Verbindung der allgemeinen Formel

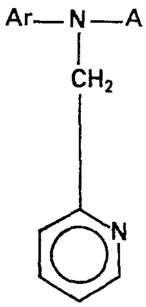

IV

in der Ar wie oben angegeben definiert ist und A eine Cyanogruppe oder den Rest

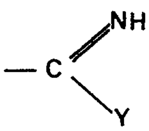

bedeutet, Y eine Alkoxy- oder Alkylthiogruppe mit bis zu 4 Kohlenstoffatomen oder eine Sulfhydryl- oder Aminogruppe darstellt, mit Äthylendiamin oder dessen Säureadditionssalzen umsetzt, und dass man gegebenenfalls das nach einem der Verfahren erhaltene Endprodukt in ein Säureadditionssalz überführt.

2. Verfahren nach Anspruch 1 a), dadurch gekennzeichnet, dass man die Umsetzung von Temperaturen von etwa 50 bis 150 °C durchführt.

3. Verfahren nach Anspruch 1 b), dadurch gekennzeichnet, dass man die Umsetzung bei einer Temperatur zwischen 60 und 180 °C durchführt.

4. Verfahren nach Anspruch 1 a) und/oder 2, dadurch gekennzeichnet, dass man die Umsetzung in Gegenwart eines polaren oder unpolaren organischen Lösungsmittels durchführt.

5. Verfahren nach Anspruch 1 a), 2 und/oder 4, dadurch gekennzeichnet, dass man die Umsetzung in Gegenwart eines säurebindenden Mittels durchführt.

6. Verfahren zur Herstellung von Arzneimitteln, dadurch gekennzeichnet, dass man eine oder mehrere Verbindungen der allgemeinen Formel I nach Anspruch 1 oder deren Säureadditionssalze mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

7. Verwendung von Verbindungen der allgemeinen Formel I nach Anspruch 1 oder von deren physiologisch verträglichen Säureadditionssalzen zur Herstellung von Arzneimitteln für die Behandlung von Herz- und Coronarerkrankungen.

**Revendications**

*pour les Etats contractants BE, CH, DE, FR, GB, IT, LU, NL, SE.*

1. 2-phénylamino-imidazolines-(2) substituées de formule générale

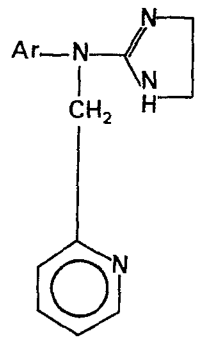

I

dans laquelle Ar représente le radical 2,6-dichlorophényle, 2,5-dichlorophényle, 2-chloro-6-méthylphényle, 2-chloro-4-méthylphényle, 2,6-dichloro-4-bromophényle, 4-cyanophényle, 2,5-diméthoxyphényle ou 2-méthyl-5-fluorophényle ainsi que leurs sels d'addition avec des acides.

2. Procédé de préparation de 2-phénylamino-imidazolines-(2) substituées de formule générale I selon la revendication 1 et de leurs sels d'addition avec des acides, caractérisé en ce que

a) on fait réagir une 2-phényl-iminoimidazolidine de formule générale

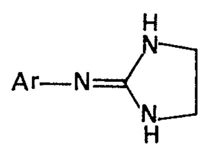

II

dans laquelle Ar possède les significations mentionnées plus haut, avec un halogénure de formule générale

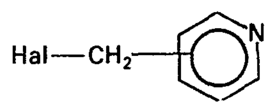

III

où Hal représente un atome de chlore, de brome ou d'iode ou bien

b) on fait réagir un composé de formule générale

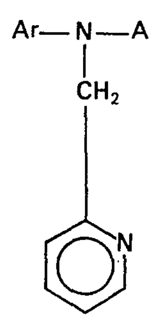

IV

dans laquelle Ar est défini comme indiqué plus haut et A représente un groupe cyano ou le radical

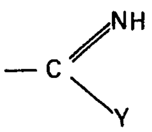 ,

où Y représente un groupe alcoxy ou alcoylthio comprenant jusqu'à 4 atomes de carbone ou un groupe sulfhydryle ou amino, avec l'éthylènediamine ou ses sels d'addition avec des acides, et en ce qu'on transforme éventuellement le produit final obtenu selon l'un des procédés en un sel d'addition avec un acide.

3. Procédé selon la revendication 2 a), caractérisé en ce qu'on effectue la réaction à des températures d'environ 50 à 150 °C.

4. Procédé selon la revendication 2 b), caractérisé en ce qu'on effectue la réaction à une température comprise entre 60 et 180 °C.

5. Procédé selon la revendication 2 a) et/ou 3, caractérisé en ce qu'on effectue la réaction en présence d'un solvant organique polaire ou non polaire.

6. Procédé selon la revendication 2 a), 3 et/ou 5, caractérisé en ce qu'on effectue la réaction en présence d'un agent fixant acides.

7. Préparations pharmaceutiques, caractérisées en ce qu'elles renferment, en tant que substance active, un ou plusieurs composés de formule générale I selon la revendication 1 ou leurs sels d'addition avec des acides.

8. Procédé de préparation de médicaments selon la revendication 7, caractérisé en ce qu'on formule un ou plusieurs composés de formule générale I selon la revendication 1 ou leurs sels d'addition avec des acides avec des adjuvants, excipients, agents de désintégration ou lubrifiants ou respectivement substances pour obtenir un effet retard galéniques habituels en comprimés, capsules, suppositoires, solutions ou poudres.

9. Composés de formule générale I selon la revendication 1 ou leurs sels d'addition avec des acides physiologiquement supportables pour l'utilisation dans le traitement des maladies du cœur et des coronaires.

10. Utilisation des composés de formule générale I selon la revendication 1 ou de leurs sels d'addition avec des acides physiologiquement supportables pour la préparation de médicaments pour le traitement des maladies du cœur et des coronaires.

**Revendications**

*pour l'Etat contractent d'AT.*

1. Procédé de préparation de 2-phénylamino-imidazolines-(2) substituées de formule générale

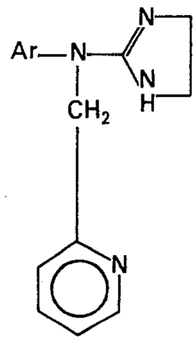

I

où Ar représente le radical 2,6-dichlorophényle, 2,5-dichlorophényle, 2-chloro-6-méthylphényle, 2-chloro-4-méthylphényle, 2,6-dichloro-4-bromophényle, 4-cyanophényle, 2,5-méthoxyphényle ou 2-méthyl-5-fluorophényle et de ses sels d'addition avec des acides, caractérisé en ce que

a) on fait réagir une 2-phényl-iminoimidazolidine de formule générale

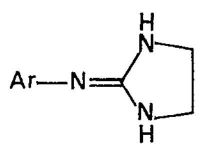

II

dans laquelle Ar possède les significations mentionnées plus haut, avec un halogénure de formule générale

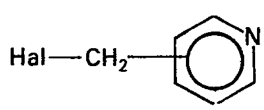

III

où Hal représente un atome de chlore, de brome ou d'iode ou bien

b) on fait rèagir un composé de formule générale

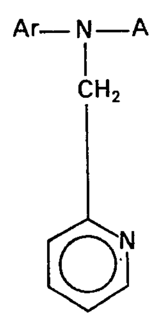

IV

dans laquelle Ar est défini comme indiqué plus haut et A représente un groupe cyano ou le radical

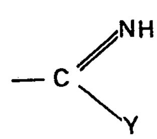 ,

où Y représente un groupe alcoxy ou alcoylthio comprenant jusqu'à 4 atomes de carbone ou un groupe sulfhydryle ou amino, avec l'éthylènedia-

mine ou ses sels d'addition avec des acides, et en ce qu'on transforme éventuellement le produit final obtenu selon l'un des procédés en un sel d'addition avec un acide.

2. Procédé selon la revendication 1 a), caractérisé en ce qu'on effectue la réaction à des températures d'environ 50 à 150 °C.

3. Procédé selon la revendication 1 b) caractérisé en ce qu'on effectue la réaction à une température comprise entre 60 et 180 °C.

4. Procédé selon la revendication 1 a) et/ou 2, caractérisé en ce qu'on effectue la réaction en présence d'un solvant organique polaire ou non polaire.

5. Procédé selon la revendication 1 a), 2 et/ou 4, caractérisé en ce qu'on effectue la réaction en présence d'un agent fixant les acides.

6. Procédé de préparation de médicaments, caractérisé en ce qu'on formule un ou plusieurs composés de formule générale I selon la revendication 1 ou leurs sels d'addition avec des acides, avec des adjuvants, excipients, agents de désintégration ou lubrifiants ou respectivement substances pour obtenir un effet retard galéniques habituels en comprimés, capsules, suppositoires, solutions ou poudres.

7. Utilisation des composés de formule générale I selon la revendication 1 ou de leurs sels d'addition avec des acides physiologiquement supportables pour la préparation de médicaments pour le traitement des maladies du cœur et des coronaires.

**Claims:**

*for the contracting states BE, CH, DE, FR, GB, IT, LU, NL, SE*

1. Substituted 2-phenylamino-imidazolines-(2) of general formula

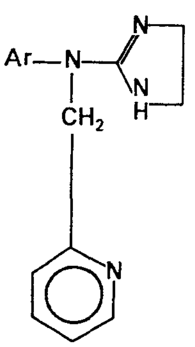

I

wherein Ar represents the group 2,6-dichlorophenyl, 2,5-dichlorophenyl, 2-chloro-6-methylphenyl, 2-chloro-4-methylphenyl, 2,6-dichloro-4-bromophenyl, 4-cyanophenyl, 2,5-dimethoxyphenyl or 2-methyl-5-fluorophenyl and the acid addition salts thereof.

2. Process for preparing substituted 2-phenylamino-imidazolines-(2) of general formula I according to claim 1 and the acid addition salts thereof, characterised in that

a) a 2-phenyl-iminoimidazolidine of general formula

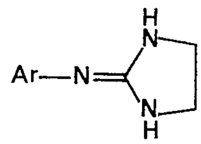

II

wherein Ar is as hereinbefore defined, is reacted with a halide of general formula

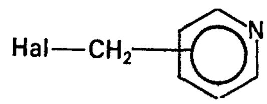

III

wherein Hal represents a chlorine, bromide or iodine atom, or

b) a compound of general formula

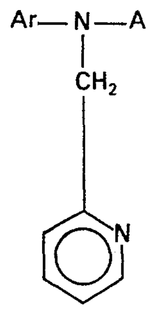

IV

wherein Ar is as hereinbefore defined and A represents a cyano group or the group

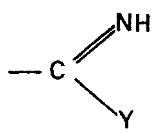

,

wherein Y represents an alkoxy or alkylthio group with up to 4 carbon atoms or a mercapto or amino group, is reacted with ethylene diamine or the acid addition salts thereof, and optionally the end product obtained according to one of these processes is converted into an acid addition salt.

3. Process acoording to claim 2 a), characterised in that the reaction is carried out at temperatures of from about 50 to 150 °C.

4. Process according to claim 2 b), characterised in that the reaction is carried out at a temperature of between 60 and 180 °C.

5. Process according to claim 2 a) and/or 3, characterised in that the reaction is carried out in the presence of a polar or apolar organic solvent.

6. Process according to claim 2 a, 3 and/or 5, characterised in that the reaction is carried out in the presence of an acid-binding agent.

7. Pharmaceutical preparations, characterised in that they contain, as active principle, one or more compounds of general formula I according to claim 1 or the acid addition salts thereof.

8. Process for preparing pharmaceutical compositions according to claim 7, characterised in that one or more compounds of general formula I as claimed in claim 1 or the acid addition salts thereof is or are formulated with conventional galenic excipients, carriers, disintegrants or lubricants or substances for obtaining sustained release, to form tablets, capsules, supositories, solutions or powders.

9. Compounds of general formula I according to claim 1 or the physiologically acceptable acid addition salts thereof for use in the treatment of heart and coronary diseases.

10. Use of compounds of general formula I according to claim 1 or the physiologically acceptable acid addition salts thereof for the preparation of pharmaceutical compositions for the treatment of heart and coronary diseases.

**Claims:**

*for the contracting state AT*

1. Process for preparing substituted 2-phenylamino-imidazolines-(2) of general formula

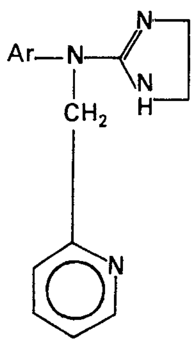

I

wherein Ar represents the group 2,6-dichlorophenyl, 2,5-dichlorophenyl, 2-chloro-6-methylphenyl, 2-chloro-4-methylphenyl, 2,6-dichloro-4-bromophenyl, 4-cyanophenyl, 2,5-dimethoxyphenyl or 2-methyl-5-fluorophenyl, and the acid addition salts thereof, characterised in that

a) a 2-phenyl-iminoimidazolidine of general formula

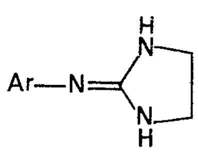

II

wherein Ar is a hereinbefore defined, is reacted with a halide of general formula

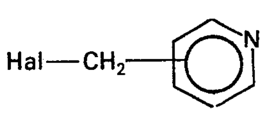

III

wherein Hal represents a chlorine, bromine or iodine atom, or

b) a compound of general formula

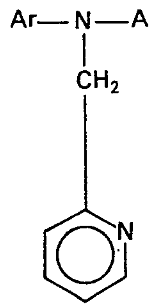

IV

wherein Ar is as hereinbefore defined and A represents a cyano group or the group

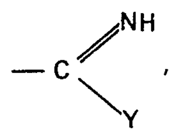

Y represents an alkoxy or alkylthio group with up to 4 carbon atoms or a mercapto or amino group, is reacted with ethylenediamine or the acid addition salts thereof, and optionally the end product obtained according to one of the processes is converted into an acid addition salt.

2. Process according to claim 1 a), characterised in that the reaction is carried out at temperatures of from about 50 to 150 °C.

3. Process according to claim 1 b), characterised in that the reaction is carried out at a temperature of between 60 and 180 °C.

4. Process according to claim 1 a) and/or 2, characterised in that the reaction is carried out in the presence of a polar or apolar organic solvent.

5. Process according to claim 1 a), 2 and/or 4, characterised in that the reaction is carried out in the presence of an acid-binding agent.

6. Process for preparing pharmaceutical compositions, characterised in that one or more compounds of general formula I according to claim 1 or the acid addition salts thereof is or are formulated with conventional galenic excipients, carriers, disintegrants or lubricants or substances for obtaining sustained release to form tablets, capsules, suppositories, solutions or powders.

7. Use of compounds of general formula I according to claim 1 or the physiologically acceptable acid addition salts thereof for the preparation of pharmaceutical compositions for the treatment of heart and coronary diseases.